# EUROPEAN PATENT SPECIFICATION

(11) **EP 0 553 272 B1**
(45) Date of publication and mention of the grant of the patent: **17.05.2000**
(21) Application number: 91920210.1
(22) Date of filing: 17.10.1991
(51) Int. Cl.: A61K 38/00, A61K 31/685

(54) **SYNTHETIC LUNG SURFACTANT**
SYNTHETISCHE OBERFLÄCHENAKTIVE AGENTIEN AUF DER LUNGE
AGENT TENSIO-ACTIF SYNTHETIQUE POUR LE TRAITEMENT DES POUMONS

(30) Priority: 17.10.1990 US 599493
(43) Date of publication of application: 04.08.1993
(73) Proprietor: THE LIPOSOME COMPANY, INC., Princeton, NJ 08540 (US)
(72) Inventor: JANOFF, Andrew, S., Yardley, PA 19067 (US)
(74) Representative: Warcoin, Jacques
(86) International application number: US9107746
(87) International publication number: WO9206703

(56) References cited:
- US-A- 4 915 951
- BIOCHIM. BIOPHYS. ACTA vol. 513, no. 1 , 1978 pages 21 - 30 P.R. CULLIS 'Effects of cholesterol on the properties of equimolar mixtures of synthetic phosphatidylethanolamine and phosphatidylcholine.'
- CLIN. PHARM vol. 8, no. 8 , 1989 pages 559 - 576 M.S. REYNOLDS 'Use of surfactant in the prevention and treatment of neonatal respiratory distress syndrome.'
- BIOCHEM. SOC. TRANSACTIONS vol. 13, no. 6 , 1985 pages 1079 - 1081 J.L. HARWOOD 'Lung surfactant'
- Biochimica et Biophysica Acta, 776 (1984), YU et al., pages 37-47. "Artifical Pulmonary Surfactant", see entire document.

## Description

### FIELD OF THE INVENTION

This invention relates to lung surfactant, and more particularly to a liposomal lung surfactant which can be used to coat lung alveoli in like manner as natural pulmonary surfactant.

### BACKGROUND OF THE INVENTION

In "Artificial Pulmonary Surfactant", Biochimica et Biophysica Acta, 776:37-47 (1984), Shou-Hwa Yu et al. describe the natural pulmonary or lung surfactants which coat the alveoli of mammalian lungs.

So do Reynolds et al. in "Use of surfactant in the prevention and treatment of neonatal respiratory distress syndrome", Clinical Pharmacy, Vol. 8 (8):559-576 (1989) and Harwood a al. in "Lung surfactant", Biochemical Society Transactions", Vol. 13(6):1079-1081(1985). Such surfactants comprise lipids and surfactant-associated proteins. More precisely, natural lung surfactant is a mixture of phosphatidylcholine, phosphatidylethanolamine, phosphatidylglycerol and proteins. Especially phosphatidylglycerol seems to be playing an essential role in these natural surfactant, and is always present (see Clin. Pharm. vol. 8 (1989) pages 559-576 and Biochem. Soc. Transactions vol. 13 (1985) pages 1079-1081). Furthermore, very small quantities of cholesterol, triglycerides and fatty acids are also present.

Pulmonary surfactant reduces surface tension at the air-liquid interface of the alveoli. Studies indicate that in the presence of surfactant, the surface tension in normally expanded lungs approximates 30 dyne/cm, while during expiration this value approaches 0 dyne/cm. Presence of pulmonary surfactant is particularly critical at birth, when the newborn infant must clear its lungs of pulmonary fluid and establish regular breathing. Absence of sufficient surfactant stores to maintain a low surface tension during the neonatal period appears to be the major factor associated with the development of the neonatal respiratory distress syndrome, also known as hyaline membrane disease, the principal cause of perinatal mortality and morbidity in developped countries. Treatment of infants suffering from advanced neonatal respiratory distress syndrome with surfactant preparations derived from bovine surfactant lipid extracts has shown a marked improvement in lung expansion and gas exchange as discussed by Reynolds a al. In like manner, a deficiency of lung surfactant in adults can cause adult respiratory distress syndrome (ARDS).

It is generally acknowledged that the ability of pulmonary surfactant to reduce the surface tension of an air/liquid interface to near 0 dyne/cm is dependent upon the formation of a monolayer of relatively pure dipalmitoyl phosphatidylcholine (DPPC) during compression. However, at normal body temperature of 37°C hydrated bilayers of DPPC exist in the gel state, from which adsorption can occur only very slowly. As a result, attempts to develop artificial mixtures which could function to transfer DPPC to the air/liquid interface at a sufficient rate to maintain normal lung functions have not been successful. One approach towards the formation of artificial surfactants has been to devise methods for dispersing DPPC using long-chain alcohols or inert hydrocarbon oils, or by administering "dry" lipid mixtures in which the DPPC is not fully hydrated.

Another possibility discussed by Yu et al., cited above, is to disperse the DPPC with other lipids which promote the formation of the hexagonal H_{II} phase. This latter state consists of elongated cylinders of lipids in inverted micellar form with the fatty acids extending outwards and the polar head-groups binding an inner core or pore of water which extends along the elongated cylinder. Because air is more hydrophobic than water, it is postulated by Yu et al. that a cylinder of H_{II} phase interacting with the air/liquid interface could tend to unfold, thereby transferring many lipid molecules to a surface. However, because of the thick gel-like consistency of hexagonal H_{II} lipids it is not believed that DPPC in such a mixture could be effectively administered in vivo to deposit a surfactant monolayer on alveoli surfaces.

When administering DPPC to alveoli as a lung surfactant, it is important that the DPPC form a monolayer spread over as much of the alveoli surface area as possible, to provide maximum treatment to the lung. Furthermore, the rate of monolayer deposition may be critical in the treatment of a subject with breathing difficulties brought on by respiratory distress syndrome. The prior methods for delivering DPPC to lung alveoli have not been able to deposit an effective surfactant monolayer of DPPC to the alveoli, the spreading rate has been found to be too slow to form an effective monolayer or to provide adequate relief for breathing difficulties. Similarly, as discussed above, the thick gel-like consistency of hexagonal HII lipids is believed to make it unsuitable for forming a monolayer on alveoli surfaces.

Cholesterol is known to have a destabilizing effect on lipid bilayers which are capable of forming the HII phase. Cullis et al. in "Effects of cholesterol on the properties of equimolar mixtures of synthetic phosphatidylethanolamine and phosphatidylcholine", Biochimica et Biophysica Acta, Vol. 513(1):21-30 (1978), observe that the addition of cholesterol with equimolar mixtures of synthetic dipalmitoyl phosphatidylcholine (DPPC) and synthetic dioleyl phosphatidylethanolamine (DOPE) surprisingly stabilizes the bilayer phase.

### SUMMARY OF THE INVENTION

In accordance with the present invention a synthetic lung surfactant composition is provided for depositing a monolayer of a phosphatidylcholine lung surfactant to the alveoli of a mammal. The composition consists of a pharmaceutically acceptable carrier suitable for pulmonary administration and a phosphatidylcholine lung surfactant, a phosphatidylethanolamine, and cholesterol in proportions such that the composition is in lamellar form at or below room temperature and in non-lamellar form at a temperature of 30°C to 37°C (a temperature at or near the body temperature of the mammal being treated). A preferred phosphatidylcholine lung surfactant for use in the present invention is dipalmitoyl phosphatidylcholine (DPPC), which is known to be the major lipid constituent of natural mammalian lung surfactant, and a preferred phosphatidylethanolamine is dioleoyl phosphatidylethanolamine (DOPE). In a particular embodiment of the present invention, the composition comprises, by molar ratio, about 2 to 4 parts DPPC and about 8 to 6 parts DOPE, for a total of 10 parts combine DPPC and DOPE, and about 5 to 10 parts cholesterol. Preferably it comprises, by mole ratio, about 3 parts DPPC to about 7 parts DOPE to about 5 to 10 parts cholesterol, all parts by molar ratio. (Unless otherwise indicated, throughout this application all parts and percentages are by molar ratio.)

In a further embodiment, the composition of the present invention may include additionally one or more therapeutic peptides, such as surfactant- associated proteins. Such proteins can be either naturally derived or synthetic. The composition may also include one or more additional pharmaceutical agents.

In accordance with a further embodiment of the invention, there is provided a pharmaceutical formulation suitable for intubation, inhalation or other means of pulmonary administration, comprising the synthetic lung surfactant composition of the present invention in association with pharmaceutical carriers or diluents.

### BRIEF DESCRIPTION OF THE DRAWINGS

Figure 1 is a graphical representation of the results of 360-MHz ³¹P-NMR spectra measured at temperatures ranging from 6 to 46°C for a sample of lung surfactant made in accordance with the present invention, comprising DOPE-DPPC-Cholesterol in a 7:3:10 molar ratio.
Figures 2A and 2B are graphical representations of the results of small-angle X-ray diffraction intensity (arbitrary units) versus inverse reciprocal distance, measured at 23°C and 38°C, for a sample of lung surfactant made in accordance with the present invention, comprising DOPE-DPPC-Cholesterol in a 7:3:5 molar ratio.
Figures 3A and 3B are graphical representations of the results of small-angle X-ray diffraction measurements at 23°C and 38°C for a comparative example, not made in accordance with the present invention, comprising DOPE-DPPC-Cholesterol in a 7:3:3 molar ratio.

### DETAILED DESCRIPTION OF THE INVENTION

The present invention stems from the discovery that when a phosphatidylcholine (PC) lung surfactant passes from bilayer form to hexagonal H_{II} or other non-bilayer form, the PC passes through a disordered intermediate phase. This intermediate phase generates monomers which spread rapidly over a hydrous surface causing a rapid reduction in surface tension. For example, the bilayer and hexagonal H_{II} forms of dipalmitoyl phosphatidylcholine (DPPC) are both very stable, while the disordered intermediate phase only exists for a brief time until the DPPC can reform into one of its stable forms. The key to the present invention is devising a means to cause the PC to pass through the monolayer transition phase while in situ at the surface of lung alveoli. If this can be achieved, then the PC will spread rapidly over the alveoli surface while in its disordered monomer form.

Certain phosphatidylethanolamines (PEs) are known to be polymorphic, having both lamellar and a hexagonal H_{II} forms. Furthermore, such PEs are known to transform from their lamellar form to their hexagonal H_{II} form with increasing temperature. (See Tilcock, "Lipid Polymorphism", Chemistry and Physics of Lipids, 40:109, 110-111 (1986).) Cholesterol is known, in general, to have a destabilizing effect on bilayer forms of lipids capable of forming H_{II} phase.

In the present invention a PC lung surfactant is combined with a pharmaceutically acceptable carrier suitable for pulmonary administrate a PE, and with a small amount of cholesterol, in suitable proportions to form a synthetic lung surfactant composition which is in stable bilayer form at room temperature and which passes through a disordered transition phase to hexagonal H_{II} or other non-lamellar form at temperature of 30°C to 37°C, (temperature at or near the body temperature of the mammal being treated).

A preferred phosphatidyleholine lung surfactant for use in the present invention is dipalmitoyl phosphatidylcholine (DPPC), which comprises two fully saturated C16 "tail" groups on a PC "head" group. Other PCs which may be used as the PC lung surfactant include dimyristoyl phosphatidylcholine (DMPC) (C14 tail groups) and distearoyl phosphatidylcholine (DSPC) (C18 tail groups).

A preferred phosphatidylethanolamine for use in the present invention is dioleoyl phosphatidylethanolamine (DOPE), which is chemically compatible with DPPC, which is biologically safe for use in the lungs, and which produces a composition with the desired temperature-dependent phase transition properties. However, any chemically compatible and biologically safe phosphatidylethanolamine which can combine with a selected phosphatidylcholine lung surfactant to give the desired temperature-dependent phase transition may be used.

For compositions comprising DPPC and DOPE, good results are obtained using these components in molar ratios of about 2 to 4 parts DPPC to about 8 to 6 parts DOPE, preferably about 3 parts DPPC to about 7 parts DOPE, to obtain the desired phase transition temperature.

The third component of the composition is cholesterol (CHOL), which is known for its effectiveness in disrupting lipid bilayers that are capable of forming HII phase. In the present composition, the cholesterol is believed to destabilize the relatively stable lamellar phase, so that the lung surfactant enters an unstable disordered intermediate phase at the desired temperature. Good results are obtained using a molar ratio of about 5 to 10 parts cholesterol to 10 parts of combined phosphatidylcholine and phosphatidylethanolamine.

The composition of the present invention may also include one or more therapeutic peptides, such as surfactant-associated surface active proteins. Such proteins can be either naturally derived or synthetic. Of particular interest are those proteins which are found in natural lung surfactants, or synthetic peptides which mimic such proteins. Some of these surfactant-associated proteins have been found to be enriched in positively charged amino acid groups, and to contain both hydrophobic and hydrophilic peptide groups.

Among the synthetic peptides which have been found useful in the lung surfactant compositions of the present invention are those comprising the peptides Arginine (abbreviated "R"), which is hydrophobic in character, and Leucine (abbreviated "L"), which is hydrophilic in character. A particlularly useful synthetic peptide for use in the surfactants of the present invention is denominated RL₄ and consists of Arginine and Leucine groups in the configuration:

R-(L)₄-R-(L)₄-R-(L)₄-R-(L)₄-R

For the tests discussed below, RL₄ peptide was obtained from the Research Institute of Scripps Clinic, La Jolla, CA.

The compositions of the present invention may also include one or more additional other pharmaceutical agents.

### EXAMPLE 1

To exhibit the temperature-dependent polymorphic properties of the composition of the present invention, a sample was made comprising DOPE-DPPC-CHOL in a molar proportion of 7:3:10, using 55.4 g of DOPE, 23.4 g of DPPC, and 41.2 g of cholesterol. The DOPE and DPPC were obtained from Avanti Polar Lipids, Birmingham, Alabama; and the cholesterol from Baker Chemicals. The material was dried to a thin film from a chloroform mixture using roto-evaporation, and suspended in 6 ml of 10 mM HEPES buffer with 150 mM sodium chloride at a pH of 7.4.

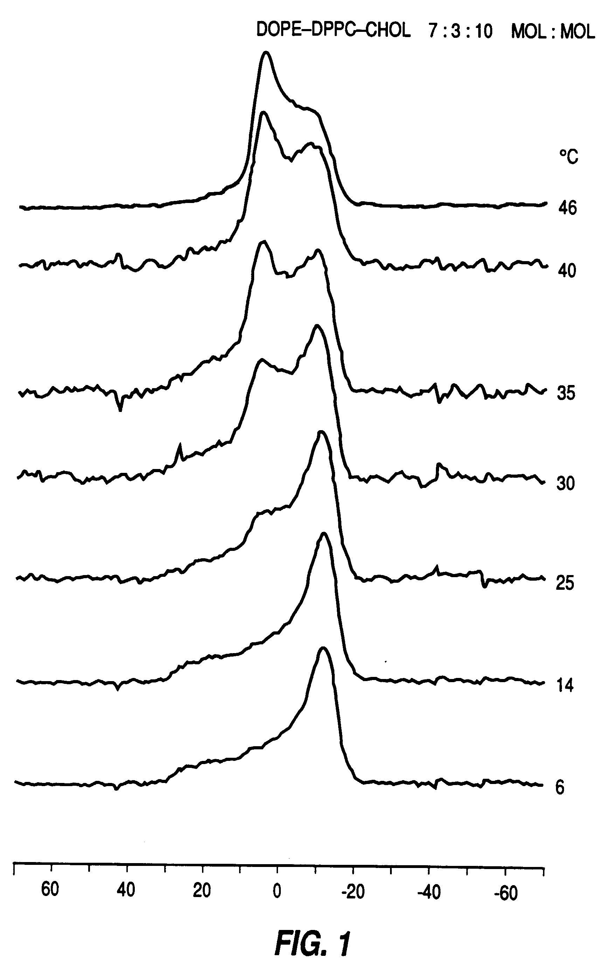

Figure 1 shows 360-MHz ³¹P-NMR spectra measured at temperatures ranging from 6 to 46°C. The change in shape of the graph lines as temperature increases is indicative of a transformation of the composition from lamellar phase to hexagonal H_{II} phase. This type of spectra data and its significance to lipid phases is discussed at length in Tilcock, cited above. At room temperature, 25°C, the composition is still in lamellar form, while at 40°C it has clearly shifted to a wave pattern characteristic of H_{II} phase. At 35°C, which is just below normal body temperature, the pattern is believed to show a transition stage, in which both lamellar and H_{II} phase are present.

### EXAMPLE 2

Two additional samples of lung surfactant compositions were made using the materials and procedures of Example 1. Sample 2 was a DOPE-DPPC-CHOL composition in a molar ratio of 7:3:5, containing 66.9 g DOPE, 28.3 g DPPC, and 24.8 g cholesterol, suspended in 6 ml buffer. Sample 3 was a DOPE-DPPC-CHOL composition in a molar ratio of 7:3:3, containing 30.4 g DOPE, 12.9 g DPPC, and 6.8 g cholesterol, suspended in 2 ml buffer.

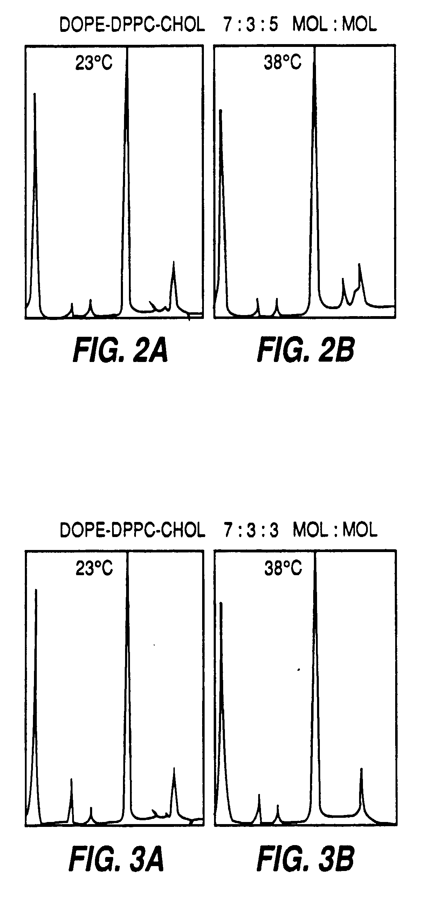

Both samples were tested by small angle X-ray diffraction, and the results are shown in Figures 2A and 2B, and 3A and 3B. In each figure, the graph A on the left represents the results for the sample at 23°C, and the graph B on the right represents the results for the sample at 38°C. The phase of a given sample can be determined by examining the right side of a given graph. Figures 2A and 3A both show a single peak at the right of the tall peak near the center of each graph. This double peak is a signature indicative of lipids in a lamellar phase. Figure 3B also shows this same signature, indicating that at 38°C Sample 3 is still in a lamellar phase. Figure 2B shows a distinct double peak at the same location on the right side of the graph. This is a signature which is at least indicative of a change out of the lamellar phase, and may represent a transition to hexagonal H_{II} phase. From these results, it may be seen that the composition containing 3 parts of cholesterol was still in the lamellar phase at a temperature of 38°C, while the composition containing 5 parts cholesterol clearly shows a transition out of the lamellar phase at or below 38°C.

### EXAMPLE 3

Tests were conducted to determine the effectiveness of the lung surfactants of the present invention in the treatment of breathing problems associated with premature birth. As discussed above, lung surfactant is particularly critical at birth, when a newborn infant must clear its lungs of pulmonary fluid and establish regular breathing. A deficiency of lung surfactant at this critical stage can result in severe breathing difficulties and even death.

In the present tests, premature fetal rabbits were used as a model of lung surfactant insufficiency, such rabbits being known to have difficulty initiating breathing due to a low level of natural lung surfactant. In each case, the fetal rabbit was administered a 0.2 ml or 0.3 ml test surfactant, ventilated, and monitored for breathing. After 30 minutes, the animal was examined for quality of breathing and color, with a healthy pink color being indicative of good breathing. In addition, lung volume and airway pressure were measured, and these results used to calculate dynamic compliance, expressed in arbitrary units as a ratio of flow to pressure per unit of body weight. The higher the value of the measured compliance, the better the breathing of the test animal. These test procedures are discussed further in Revak, et al., "Reconstitution of surfactant activity using purified human apoprotein and phospholipids measured in vitro and in vivo", Am. Rev. Respir. Dir. 134:1258-1265 (1986).

Tests were conducted using 7:3:10 DOPE-DPPC-Cholesterol surfactant made in accordance with Example 1, above (Sample A), and 7:3:5 DOPE-DPPC-Cholesterol surfactant made in accordance with Example 2 (Sample B).

As discussed above, the compositions of the present invention can also include one or more therapeutic proteins. Lung surfactants in accordance with the present invention were made from soy phosphatidylcholine (soy PE), DPPC, and cholesterol in combination with the peptide RL₄, discussed above. Samples of peptide-containing surfactants were prepared comprising soy PE-DPPC-cholesterol in a 7:3:3 ratio and containing 3% by weight RL₄ peptide (Sample C), and soy PE-DPPC-cholesterol in a 7:3:5 ratio, containing 3% by weight RL₄ peptide (Sample D). Samples C and D were also included in the present fetal rabbit model tests.

Additional tests were conducted using 150 mM saline solution as a negative control, and native human surfactant, isolated from term amniotic fluid, as a positive control.

Three set of tests were conducted using the example surfactants and the negative and positive controls. In the first two sets of tests, 0.2 ml doses were administered, while in the third set of tests 0.3 ml doses were used. In the tests in which the animals were administered example surfactants Samples A, B, C, or D, or the human surfactant positive control, all showed excellent breathing within 2 to 3 minutes of ventilation, and all acquired a healthy pink color. At the end of the 30-minute test periods, all of these animals continued to show good breathing and healthy pink color. The rabbits administered the saline negative control had difficulty breathing, and did not have a healthy pink color after 30 minutes. These tests demonstrated that example surfactants Samples A, B, C and D, all made in accordance with the present invention, exhibited comparable results to native human surfactant in treating the respiratory problems associated with an insufficiency of natural lung surfactant.

Compliance values for each animal were measured at periodic intervals over the course of the 30-minute tests. The compliance results are presented in Table 1, with the compliances expressed in arbitrary units as discussed above. The compliance values of the animals administered the example surfactants Samples A, B, C, and D, and the human surfactant positive controls were all at least 25-300% higher that those of the saline negative control animals.

In accordance with another aspect of the present invention, a pharmaceutical formulation suitable for intubation, inhalation or other means of pulmonary administration, comprises the synthetic lung surfactant composition of the present invention in association with appropriate pharmaceutical carriers or diluents. A process for the manufacture of such a pharmaceutical formulation comprises combining phosphatidylcholine lung surfactant, phosphatidylethanolamine, and cholesterol to form the artificial lung surfactant composition of the present invention, and mixing the thus-formed composition with the pharmaceutical carriers or diluents.

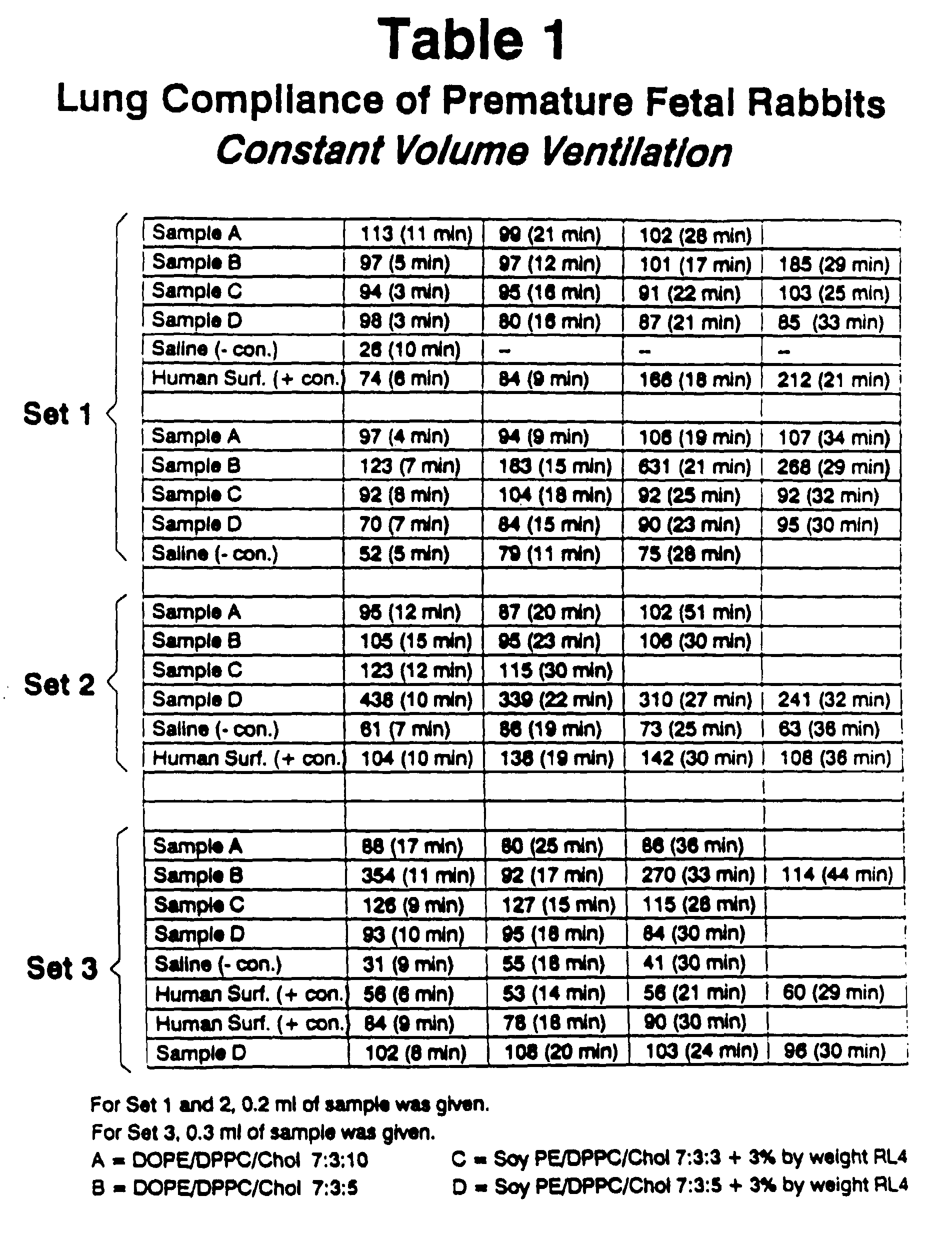

## Claims

1. A synthetic lung surfactant composition consisting of a pharmaceutically acceptable carrier suitable for pulmonary administration and a phosphatidylcholine lung surfactant, a phosphatidylethanolamine, and cholesterol in proportions such that the composition is in lamellar form at or below room temperature and in non-lamellar form at a temperature of 30° C to 37° C.

2. The composition of claim 1, wherein the phosphatidylcholine lung surfactant is dipalmitoyl phosphatidylcholine (DPPC) and the phosphatidylethanolamine is dioleoyl phosphatidylethanolamine (DOPE).

3. The composition of claim 2 comprising, by molar ratio, about 2 to 4 parts DPPC and about 8 to 6 parts DOPE, for a total of 10 parts combined DPPC and DOPE, and about 5 to 10 parts cholesterol.

4. The composition of claim 3 comprising, by molar ratio, about 3 parts DPPC, about 7 parts DOPE, and about 5 to 10 parts cholesterol.

5. The composition of claim 4 comprising, by molar ratio, about 3 parts DPPC, about 7 Parts DOPE and 5 parts cholesterol.

6. The composition of claim 1, which additionally includes one or more therapeutic peptides.

7. A pharmaceutical formulation suitable for intubation, inhalation or other means of pulmonary administration, said formulation comprising the synthetic lung surfactant composition of claim 1.

## Patentansprüche

1. Eine synthetische Lungensurfactant-Zusamnensetzung, bestehend aus einem pharmazeutisch annehmbaren Träger, der zur pulmonalen Verabreichung geeignet ist und einem Phosphatidylcholin-Lungensurfactant, einem Phosphatidylethanolamin und Cholesterol in solchen Anteilen, dass die Zusammensetzung bei oder unterhalb der Raumtemperatur in lamellarer Form und bei einer Temperatur von 30 °C bis 37 °C in nicht-lamellarer Form vorliegt.

2. Die Zusammensetzung gemäß Anspruch 1, worin das Phosphatidylcholin-Lungensurfactant Dipalmitoylphosphatidylcholin (DPPC) ist und das Phosphatidylethanolamin Dioleoylphosphatidylethanolamin (DOPE) ist.

3. Die Zusammensetzung gemäß Anspruch 2, umfassend, im Molverhältnis, etwa 2 bis 4 Teile DPPC und etwa 8 bis 6 Teile DOPE, bezüglich einer Gesamtmenge von 10 Teilen kombinierten DPPC und DOPE und etwa 5 bis 10 Teilen Cholesterol.

4. Die Zusammensetzung gemäß Anspruch 3, umfassend, im Molverhältnis, etwa 3 Teile DPPC, etwa 7 Teile DOPE und etwa 5 bis 10 Teile Cholesterol.

5. Die Zusammensetzung gemäß Anspruch 4, umfassend, im Molverhältnis, etwa 3 Teile DPPC, etwa 7 Teile DOPE und 5 Teile Cholesterol.

6. Die Zusammensetzung gemäß Anspruch 1, welche zusätzlich eines oder mehrere therapeutische Peptide umfasst.

7. Eine pharmazeutische Formulierung, die für die Intubation, Inhalation oder andere Mittel pulmonaler Verabreichung geeignet ist, wobei besagte Formulierung die synthetische Lungensurfactant-Zusammensetzung des Anspruchs 1 umfasst.

## Revendications

1. Composition de surfactant pulmonaire synthétique, consistant en un véhicule pharmaceutiquement acceptable approprié pour l'administration pulmonaire et un surfactant pulmonaire de type phosphatidylcholine, une phosphatidyléthanolamine et du cholestérol, en proportions telles que la composition est sous forme lamellaire à ou au-dessous de la température ambiante et sous forme non lamellaire à une température de 30 à 37°C.

2. Composition de la revendication 1, dans laquelle le surfactant pulmonaire de type phosphatidylcholine est la dipalmitoylphosphatidylcholine (DPPC) et la phosphatidyléthanolamine est la dioléoylphosphatidyléthanolamine (DOPE).

3. Composition de la revendication 2, comprenant, en rapport molaire, environ 2 à 4 parties de DPPC et environ 8 à 6 parties de DOPE, pour un total de 10 parties de DPPC et DOPE réunies, et environ 5 à 10 parties de cholestérol.

4. Composition de la revendication 3, comprenant, en rapport molaire, environ 3 parties de DPPC, environ 7 parties de DOPE, et environ 5 à 10 parties de cholestérol.

5. Composition de la revendication 4, comprenant, en rapport molaire, environ 3 parties de DPPC, environ 7 parties de DOPE, et 5 parties de cholestérol.

6. Composition de la revendication 1, qui comprend en outre un ou plusieurs peptides thérapeutiques.

7. Formulation pharmaceutique appropriée à l'intubation, l'inhalation ou d'autres moyens d'administration pulmonaire, ladite formulation comprenant la composition de surfactant pulmonaire synthétique de la revendication 1.
